(19) Europäisches Patentamt
European Patent Office
Office européen des brevets

(11) Veröffentlichungsnummer: **0 041 671**
**A1**

(12) # EUROPÄISCHE PATENTANMELDUNG

(21) Anmeldenummer: **81104158.1**

(22) Anmeldetag: **01.06.81**

(51) Int. Cl.$^3$: **C 07 C 49/175**
C 07 C 49/213, C 07 C 49/233
C 07 C 49/255, C 07 C 119/042
C 07 C 149/10, C 07 C 149/30
C 07 C 149/34, C 07 C 147/06
C 07 C 161/04, C 07 D 233/58

(30) Priorität: **07.06.80 DE 3021516**

(43) Veröffentlichungstag der Anmeldung:
**16.12.81 Patentblatt 81/50**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Krämer, Wolfgang, Dr.**
**Am Eckbusch 39/45**
**D-5600 Wuppertal 1(DE)**

(72) Erfinder: **Elbe, Hans-Ludwig, Dr.**
**Dasnoeckel 59**
**D-5600 Wuppertal 11(DE)**

(54) **4-Substituierte 3,3-Dimethyl-butan-2-one, Verfahren zu ihrer Herstellung sowie ihre Verwendung als Zwischenprodukte.**

(57) Die erfindung betrifft neue 4-substituierte 3,3-Dimethyl-butan-2-one, die als Zwischenprodukte für die Synthese von Pflanzenschutzmitteln verwendet werden können, sowie mehrere Verfahren zu deren Herstellung.

Die Verbindungen der allgemeinen Formel

$$CH_3-CO-\underset{CH_3}{\overset{CH_3}{C}}-CH_2-R \qquad (I)$$

in welcher R die in der Beschreibung angegebene Bedeutung besitzt, werden erhalten, wenn man Butan-2-on-Derivate mit Verbindungen der Formel

$$R^3 - Me \qquad (III)$$

in welcher $R^3$ und Me die in der Beschreibung angegebene Bedeutung besitzen, in Gegenwart eines Verdünnungsmittels und gegebenenfalls in Gegenwart eines Säurebindemittels umsetzt; außerdem gibt es noch andere Verfahrensweisen.

Die neuen 4-substituierten 3,3-Dimethyl-butan-2-one der Formel (I) istellen interessante Zwischenprodukte dar. Sie lassen sich leicht in Halogenketone überführen, indem man die Verbindungen der Formel (I) in einem interten organischen Lösungsmittel bei Raumtemperatur mit Chlor oder Brom versetzt; die Halogenketone lassen sich weiterhin mit Phenolen umsetzen, wobei man zu Etherketonen gelangt, die anschließend durch weitere Halogenierung in Halogenetherketone überführt werden können, die sich mit Azolen glatt zu Verbindungen mit fungizider Wirksamkeit umsetzen lassen.

EP 0 041 671 A1

Croydon Printing Company Ltd.

0041671

BAYER AKTIENGESELLSCHAFT
Zentralbereich
Patente, Marken und Lizenzen

5090 Leverkusen, Bayerwerk
Slr/W
Ib/ZP

# 4-substituierte 3,3-Dimethyl-butan-2-one, Verfahren zu ihrer Herstellung sowie ihre Verwendung als Zwischenprodukte

Die vorliegende Erfindung betrifft neue 4-substituierte 3,3-Dimethyl-butan-2-one, die als Zwischenprodukte für die Synthese von Pflanzenschutzmitteln verwendet werden können, sowie mehrere Verfahren zu deren Herstellung.

Es wurden als neue Verbindungen die 4-substituierten 3,3-Dimethyl-butan-2-one der allgemeinen Formel

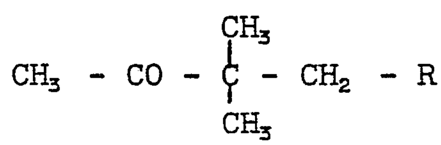

(I)

in welcher

R für die Gruppierung $-X-R^1$ oder Cyano steht, wobei

$R^1$ für n-Alkyl mit 1 bis 4 Kohlenstoffatomen, Isopropyl, Isobutyl, sek.-Butyl, Alkenyl mit 3 bis 4 Kohlenstoffatomen, Alkinyl mit 3 bis 4 Kohlenstoffatomen, gegebenenfalls substituiertes Aryl oder substituiertes Aralkyl steht, sowie auch für Cyano steht, wenn X für -O- oder -S- steht und

X für -O-, -S-, -SO- oder $-SO_2-$ steht,

gefunden.

Weiterhin wurde gefunden, daß man die 4-substituierten 3,3-Dimethyl-butan-2-one der Formel (I) erhält, wenn man

a) Butan-2-on-Derivate der Formel

$$CH_3 - CO - \underset{CH_3}{\overset{CH_3}{\underset{|}{\overset{|}{C}}}} - CH_2 - Y \qquad (II)$$

in welcher

Y für Chlor, Brom oder die Gruppierung $-O-SO_2-R^2$ steht, wobei

$R^2$ für Alkyl oder gegebenenfalls substituiertes Aryl steht,

mit Verbindungen der Formel

$R^3 - Me$ (III)

in welcher

$R^3$ für die Gruppierung $-X^1-R^4$ oder Cyano steht,

$R^4$ für n-Alkyl mit 1 bis 4 Kohlenstoffatomen, Isopropyl, Isobutyl, sek.-Butyl, Alkenyl und Alkinyl mit jeweils 3 bis 4 Kohlenstoffatomen, gegebenenfalls substituiertes Aryl, substituiertes Aralkyl oder Cyano steht,

$X^1$ für Sauerstoff oder Schwefel steht und

Me für ein Alkalimetall oder Wasserstoff steht,

in Gegenwart eines Verdünnungsmittels und gegebenenfalls in Gegenwart eines Säurebindemittels umsetzt, oder

b) 3,3-Dimethyl-4-hydroxy-butan-2-on der Formel

$$CH_3 - CO - C(CH_3)_2 - CH_2 - O - H \quad (IV)$$

mit Verbindungen der Formel

$Y^1 - R^5$ (V)

in welcher

$R^5$ für n-Alkyl mit 1 bis 4 Kohlenstoffatomen, Isopropyl, Isobutyl, sek.-

Butyl, Alkenyl und Alkinyl mit jeweils 3 bis 4 Kohlenstoffatomen oder substituiertes Aralkyl steht,

Y' für Chlor, Brom oder die Gruppierung $-O-SO_2-R^6$ steht und

$R^6$ für Alkyl, Alkoxy oder gegebenenfalls substituiertes Aryl steht,

gegebenenfalls in Gegenwart eines Verdünnungsmittels und gegebenenfalls in Gegenwart eines Säurebindemittels umsetzt, und

c) gegebenenfalls die nach dem Verfahren (a) erhältlichen Verbindungen der Formel

$$CH_3 - CO - \overset{CH_3}{\underset{CH_3}{C}} - CH_2 - S - R^7 \qquad (Ia)$$

in welcher

$R^7$ für n-Alkyl mit 1 bis 4 Kohlenstoffatomen, Isopropyl, Isobutyl, sek.-Butyl, Alkenyl und Alkinyl mit jeweils 3 bis 4 Kohlenstoffatomen, gegebenenfalls substituiertes Aryl oder substituiertes Aralkyl steht,

in üblicher Weise mit einem Oxidationsmittel umsetzt.

Die erfindungsgemäßen Stoffe sind durch die allgemeine Formel (I) eindeutig definiert. In dieser Formel steht <u>R</u> vorzugsweise für die Gruppierung $-X-R^1$ sowie Cyano. <u>$R^1$</u> steht vorzugsweise für n-Alkyl mit 1 bis 4 Kohlenstoffatomen, Isopropyl, Isobutyl, sek.-Butyl; für Alkenyl und Alkinyl mit jeweils 3 bis 4 Kohlenstoffatomen; sowie

für gegebenenfalls substituiertes Aryl mit 6 bis 10 Kohlenstoffatomen und substituiertes Aralkyl mit 6 bis 10 Kohlenstoffatomen im Arylteil und 1 bis 2 Kohlenstoffatomen im Alkylteil, wobei als Arylsubstituenten vorzugsweise genannt seien: Halogen, Cyano, Nitro, Alkoxycarbonyl mit 1 bis 4 Kohlenstoffatomen im Alkylteil, Alkyl und Alkoxy mit jeweils 1 bis 4 Kohlenstoffatomen, Dialkylamino mit 1 bis 2 Kohlenstoffatomen in jedem Alkylteil sowie gegebenenfalls durch Halogen substituiertes Phenyl. $\underline{R}^1$ steht auch vorzugsweise für Cyano, wenn X für Sauerstoff oder Schwefel steht. $\underline{X}$ hat vorzugsweise die in der Efindungsdefinition angegebene Bedeutung.

Besonders bevorzugt sind diejenigen Verbindungen der Formel (I), in denen $\underline{R}$ und $\underline{X}$ die in der Erfindungsdefinition angegebene Bedeutung haben, und $\underline{R}^1$ für Methyl, Ethyl, n-Propyl, Isopropyl, n-Butyl, Isobutyl, sek.-Butyl, Allyl, Propargyl, sowie für gegebenenfalls substituiertes Phenyl und substituiertes Benzyl steht, wobei als Substituenten infrage kommen: Fluor, Chlor, Brom, Cyano, Nitro, Methoxycarbonyl, Methyl, Ethyl, Isopropyl, tert.-Butyl, Methoxy, Dimethylamino sowie gegebenenfalls durch Fluor und Chlor substituiertes Phenyl; sowie auch für Cyano steht, wenn X für Sauerstoff oder Schwefel steht,

Verwendet man beispielsweise 3,3-Dimethyl-4-tosyloxybutan-2-on und das Natriumsalz von 4-Chlorphenol als Ausgangsstoffe, so kann der Reaktionsablauf durch das folgende Formelschema wiedergegeben werden (Verfahren a):

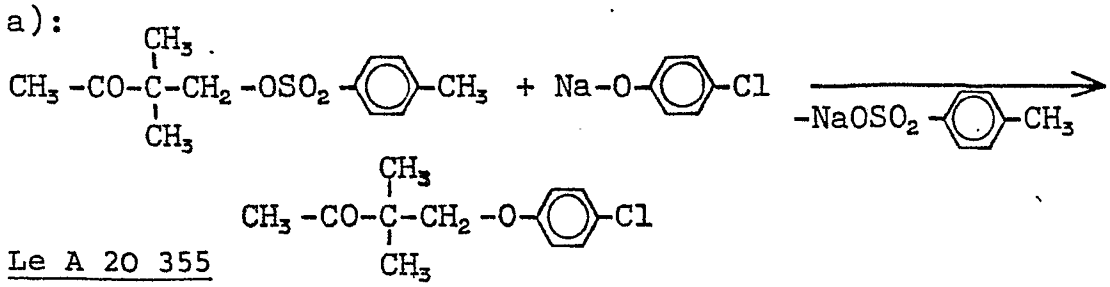

Verwendet man beispielsweise 4-Chlorpinakolin und Thiophenol als Ausgangsstoffe, so kann der Reaktionsablauf durch das folgende Formelschema wiedergegeben werden (Verfahren a):

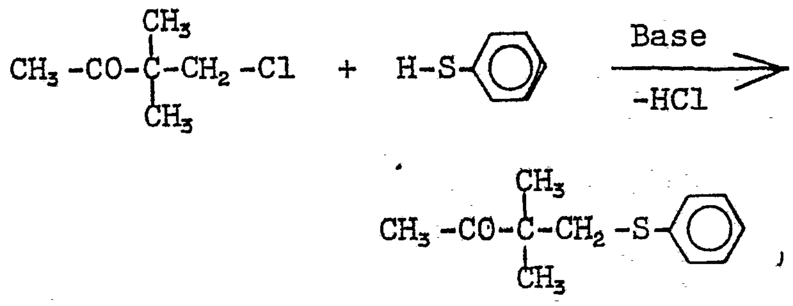


Verwendet man beispielsweise 4-Hydroxypinakolin und Dimethylsulfat als Ausgangsstoffe, so kann der Reaktionsablauf durch das folgende Formelschema wiedergegeben werden (Verfahren b):

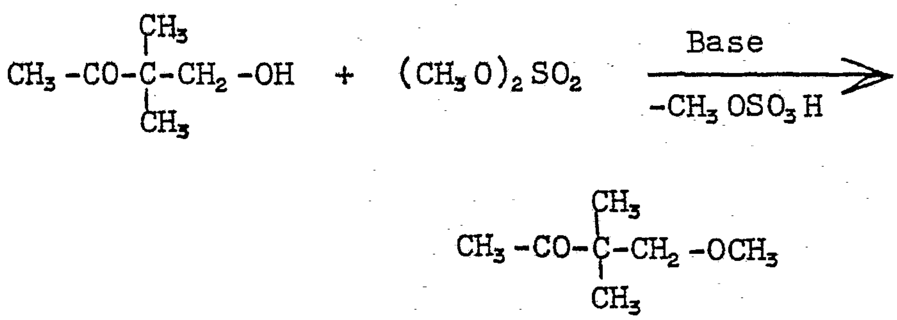


Verwendet man beispielsweise 3,3-Dimethyl-4-phenylmercapto-butan-2-on als Ausgangsstoff und Wasserstoffperoxid /Eisessig als Oxidationsmittel, so kann der Reaktionsablauf durch das folgende Formelschema wiedergegeben werden (Verfahren c):

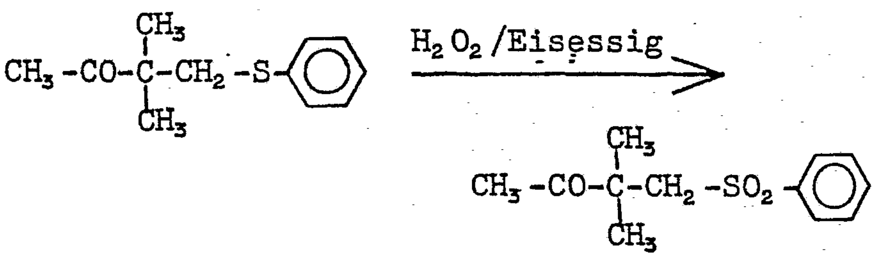

Die für das erfindungsgemäße Verfahren (a) als Ausgangsstoffe zu verwendenden Butan-2-on-Derivate sind durch die Formel (II) allgemein definiert. In dieser Formel steht Y vorzugsweise für Chlor, Brom oder die Gruppierung $-O-SO_2-R^2$, wobei $R^2$ vorzugsweise für Alkyl mit 1 bis 4 Kohlenstoffatomen sowie für gegebenenfalls substituiertes Aryl mit 6 bis 10 Kohlenstoffatomen, wie insbesondere Phenyl, steht, wobei als Substituenten vorzugsweise Alkyl mit 1 bis 4 Kohlenstoffatomen infrage kommt.

Die Butan-2-on-Derivate der Formel (II) sind bekannt (vergleiche z.B. DE-OS 26 32 603 [LeA 17 273] und J.Org. Chem. 35, 2391 (1970)), bzw. können sie in allgemein bekannter Art und Weise aus dem 3,3-Dimethyl-4-hydroxy-butan-2-on erhalten werden (vergleiche auch die Herstellungsbeispiele).

Die außerdem für das erfindungsgemäße Verfahren (a) als Ausgangsstoffe zu verwendenden Verbindungen sind durch die Formel (III) allgemein definiert. In dieser Formel steht $R^3$ vorzugsweise für die Gruppierung $-X^1-R^4$ und Cyano. $X^1$ steht vorzugsweise für Sauerstoff oder Schwefel. $R^4$ steht vorzugsweise für n-Alkyl mit 1 bis 4 Kohlenstoffatomen, Isopropyl, Isobutyl, sek.-Butyl; für Alkenyl und Alkinyl mit jeweils 3 bis 4 Kohlenstoffatomen; für Cyano; sowie für gegebenenfalls substituiertes Phenyl und substituiertes Benzyl, wobei als Substituenten infrage kommen: Halogen, Cyano, Nitro, Alkoxycarbonyl mit 1 bis 4 Kohlenstoffatomen im Alkylteil, Alkyl und Alkoxy mit jeweils 1 bis 4 Kohlenstoffatomen, Dialkylamino mit 1 bis 2 Kohlenstoffatomen in jedem Alkylteil, sowie gegebenenfalls durch Halogen substituiertes Phenyl. Me steht vorzugsweise für Kalium, Natrium und Wasserstoff.

Die Verbindungen der Formel (III) sind allgemein bekannte Verbindungen der organischen Chemie. Gegebenenfalls werden sie in-situ eingesetzt.

Das für das erfindungsgemäße Verfahren (b) als Ausgangsstoff zu verwendende 3,3-Dimethyl-4-hydroxy-butan-2-on der Formel (IV) ist bekannt (vergleiche z.B. DE-OS 26 32 603..).

Die außerdem für das erfindungsgemäße Verfahren (b) als Ausgangsstoffe zu verwendenden Verbindungen sind durch die Formel (V) allgemein definiert. In dieser Formel steht $R^5$ vorzugsweise für n-Alkyl mit 1 bis 4 Kohlenstoffatomen, Isopropyl, Isobutyl, sek.-Butyl; für Alkenyl und Alkinyl mit jeweils 3 bis 4 Kohlenstoffatomen; sowie für gegebenenfalls substituiertes Benzyl, wobei als Substituenten vorzugsweise die bereits weiter oben genannten Substituenten infrage kommen.
$Y'$ steht vorzugsweise für Chlor, Brom oder die Gruppierung $-O-SO_2-R^6$, wobei $R^6$ vorzugsweise für Alkyl und Alkoxy mit jeweils 1 bis 4 Kohlenstoffatomen sowie für gegebenenfalls durch Alkyl mit 1 bis 4 Kohlenstoffatomen substituiertes Phenyl steht.

Die Verbindungen der Formel (V) sind allgemein bekannte Verbindungen der organischen Chemie.

Die für das erfindungsgemäße Verfahren (c) als Ausgangsstoffe zu verwendenden Verbindungen der Formel (Ia) sind erfindungsgemäße Stoffe.

Als Verdünnungsmittel kommen für das erfindungsgemäße Verfahren (a) vorzugsweise organische Lösungsmittel infrage. Hierzu gehören vorzugsweise Ether, wie Diethylether und Dioxan; Alkohole, wie Glykol; aliphatische und aromatische Kohlenwasserstoffe, wie Ligroin. Petrolether, Benzol,

Toluol oder Xylol; sowie Formamide, wie Dimethylformamid.

Das erfindungsgemäße Verfahren (a) wird gegebenenfalls in Gegenwart eines Säurebindemittels durchgeführt. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat; Alkalialkoholate, beispielsweise Natriummethylat und Kaliummethylat; oder Alkalihydroxide, beispielsweise Natriumhydroxid und Kaliumhydroxid.

Die Reaktionstemperaturen können bei der Durchführung des erfindungsgemäßen Verfahrens (a) in einem größeren Bereich variiert werden. Im allgemeinen arbeitet man zwischen 80 und 200°C, vorzugsweise zwischen 80 und 150°C.

Das erfindungsgemäße Verfahren (a) wird gegebenenfalls unter erhöhtem Druck durchgeführt. Im allgemeinen arbeitet man zwischen 1 und 10 bar.

Bei der Durchführung des erfindungsgemäßen Verfahrens (a) setzt man auf 1 Mol Butan-2-on-Derivat der Formel (II) vorzugsweise etwa 1 bis 2 Mol der Verbindung der Formel (III) und gegebenenfalls etwa 1 bis 2 Mol Säurebinder ein. Die Isolierung der Verbindungen der Formel (I) erfolgt in üblicher Weise.

Als Verdünnungsmittel für das erfindungsgemäße Verfahren (b) kommen vorzugsweise Wasser und organische Lösungsmittel infrage. Hierzu gehören beispielsweise die beim Verfahren (a) bereits genannten Solventien.

Als Säurebindemittel kommen für das erfindungsgemäße Verfahren (b) vorzugsweise die beim Verfahren (a) bereits genannten Verbindungen infrage.

Die Reaktionstemperaturen können bei der Durchführung des erfindungsgemäßen Verfahrens (b) in einem größeren Bereich variiert werden. Im allgemeinen arbeitet man zwischen 0 und 120°C,vorzugsweise zwischen 20 und 100°C.

Bei der Duchführung des erfindungsgemäßen Verfahrens (b) setzt man auf 1 Mol 3,3-Dimethyl-4-hydroxy-butan-2-on der Formel (IV) vorzugsweise etwa 1 bis 2 Mol der Verbindung der Formel (V) und gegebenenfalls etwa 1 bis 2 Mol Säurebinder ein. Die Isolierung der Verbindungen der Formel (I) erfolgt in üblicher Weise.

Als Oxidationsmittel kommen für das erfindungsgemäße Verfahren (c) alle üblicherweise verwendbaren anorganischen und organischen Oxidationsmittel in Betracht. Hierzu gehören vorzugsweise organische Persäuren, wie z.B. Peressigsäure, p-Nitroperbenzoesäure, m-Chlorperbenzoesäure; anorganische Persäuren, wie z.B. Perjodsäure, weiterhin Wasserstoffperoxid in Eisessig oder Methanol, Kaliumpermanganat und Chromsäure.

Die Reaktionstemperaturen können bei der Durchführung des erfindungsgemäßen Verfahrens (c) in einem größeren Bereich variiert werden. Im allgemeinen arbeitet man zwischen etwa -50 bis 100°C, vorzugsweise zwischen -30 und 80°C.

Bei der Durchführung des erfindungsgemäßen Verfahrens (c) setzt man auf 1 Mol der erfindungsgemäßen Verbindung der Formel (Ia) etwa 1 bis 5 Mol Oxidationsmittel ein. Bei der Anwendung von 1 Mol Oxidationsmittel, wie m-Chlorperbenzoesäure in Methylenchlorid oder Wasserstoffperoxid in Acetanhydrid bei Temperaturen zwischen -30 bis +30°C, entstehen vorzugsweise die erfindungsgemäßen Verbindungen der Formel (I) mit X=SO. Bei

Ueberschuß an Oxidationsmittel und höheren Temperaturen (10 bis 80°C) entstehen vorzugsweise die erfindungsgemäßen Verbindungen der Formel (I) mit $X=SO_2$. Die Isolierung der Oxidationsprodukte erfolgt in üblicher Weise.

Wie schon erwähnt, stellen die neuen 4-substituierten 3,3-Dimethyl-butan-2-one der Formel (I) interessante Zwischenprodukte dar. Sie lassen sich leicht in Halogenketone der Formel

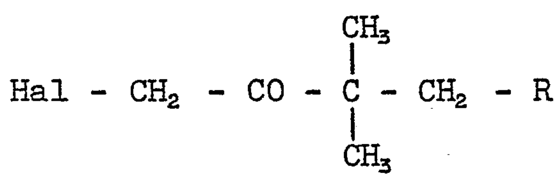 (VI)

in welcher

R die oben angegebene Bedeutung hat und

Hal für Chlor oder Brom steht,

überführen, indem man die Verbindungen der Formel (I) in einem inerten organischen Lösunsgmittel bei Raumtemperatur mit Chlor oder Brom versetzt, oder z.B. mit üblichen Chlorierungsmitteln, wie z.B. Sulfurylchlorid, bei 20 bis 60°C umsetzt.

Die Halogenketone der Formel (VI) lassen sich weiterhin mit Phenolen umsetzen (vergleiche hierzu z.B. die Angaben in der DE-OS 26 32 603 [LeA 17 273]), wobei man zu den Etherketonen der Formel

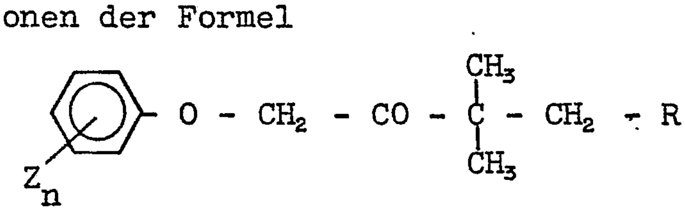 (VII)

in welcher

R die oben angegebene Bedeutung hat,

Z für Halogen, Alkyl, Alkoxy, Nitro, Cyano, Alkoxycarbonyl oder gegebenenfalls substituiertes Phenyl steht und

n für 0,1,2 oder 3 steht,

gelangt. Die Etherketone der Formel (VII) können durch weitere Halogenierung, vorzugsweise mit Sulfurylchlorid oder mit Brom, in die Halogenetherketone der Formel

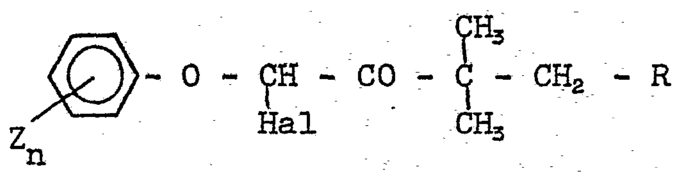 (VIII)

in welcher

Hal,R,Z und n die oben angegebene Bedeutung haben,

überführt werden, die sich mit Azolen glatt zu den Verbindungen der Formel

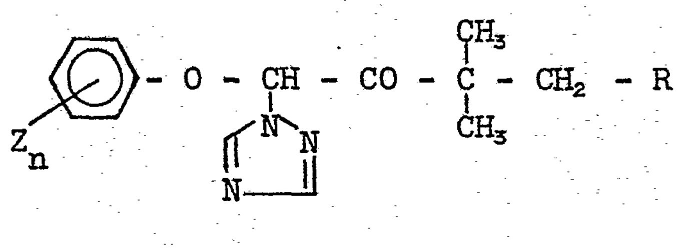 (IX)

in welcher

A für ein Stickstoffatom oder die CH-Gruppe steht und

R,Z und n die oben angegebene Bedeutung haben ,

umsetzen lassen.

Die Verbindungen der Formel (IX) weisen starke fungizide Eigenschaften auf und sind daher als Pflanzenschutzmittel verwendbar.

Der nachfolgende Vergleichsversuch zeigt z.B. die überlegene Wirkung des 1,4-Bis-(4-chlorphenoxy)-3,3-dimethyl-1-(imidazol-1-yl)butan-2-ons im Vergleich mit der aus der DE-OS 26 35 666 [LeA 17 324] bekannten, chemisch sehr naheliegenden Verbindung 1-(4-Chlorphenoxy)-4-dichloracetoxy-3,3-dimethyl-1-(imidazol-1-yl)-butan-2-on auf:

Beispiel A

Erysiphe-Test (Gurken)/ Protektiv

Lösungsmittel: 4,7 Gewichtsteile Aceton

Emulgator: 0,3 Gewichtsteile Alkyl-aryl-polyglykolether

Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.
Mit der Spritzflüssigkeit bespritzt man junge Gurkenpflanzen mit etwa drei Laubblättern bis zur Tropfnässe. Die Gurkenpflanzen verbleiben zur Trocknung 24 Stunden im Gewächshaus. Dann werden sie zur Inokulation mit Konidien des Pilzes Erysiphe cichoreacearum bestäubt. Die Pflanzen werden anschließend bei 23 bis 24°C und bei einer relativen Luftfeuchtigkeit von ca 75 % im Gewächshaus aufgestellt.
Nach 12 Tagen wird der Befall der Gurkenpflanzen bestimmt. Die erhaltenen Boniturwerte werden in Prozent Befall umgerechnet. 0 % bedeutet keinen Befall, 100 % bedeutet, daß die Pflanzen vollständig befallen sind.

Wirkstoffe, Wirkstoffkonzentrationen und Ergebnisse gehen aus der nachfolgenden Tabelle hervor:

Tabelle A

Erysiphe-Test (Gurken) / Protektiv

| Wirkstoff | Befall in % bei einer Wirkstoffkonzentration von 0,0005 % |
|---|---|
| 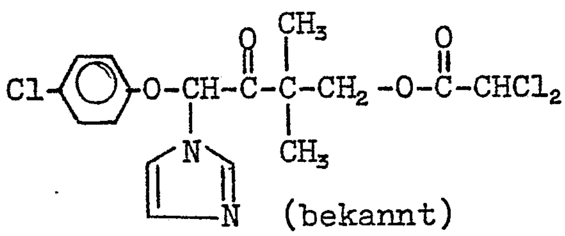 (bekannt) | 100 |
| 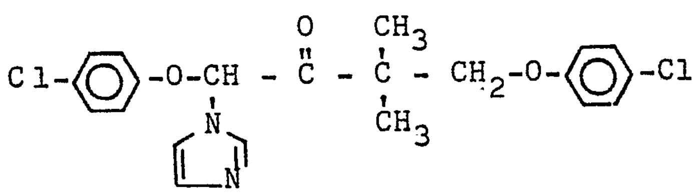 | 12 |

Herstellungsbeispiele

Beispiel 1

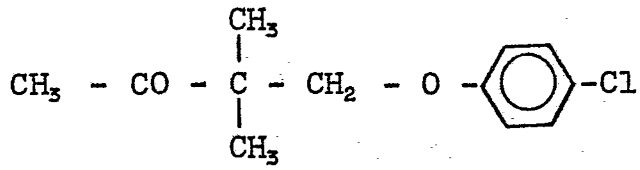

(Verfahren a)

29,7g (0,55 Mol) Natriummethylat werden in 500 ml Methanol gelöst und unter Rühren mit 70,4g (0,55 Mol) 4-Chlorphenol versetzt. Nach 10 Minuten Rühren wird das Lösungsmittel unter vermindertem Druck abdestilliert und der Rückstand in 100 ml Glykol aufgenommen. Diese Lösung wird zu einer Lösung von 135g (0,5 Mol) 2,2-Dimethyl-1-tosyloxy-butan-3-on in 200 ml Glykol gegeben. Man läßt 48 Stunden bei 100 bis 120°C rühren, kühlt ab und rührt das Reaktionsgemisch in 2000 ml Wasser ein. Man extrahiert zweimal mit je 250 ml Diethylether, wäscht die vereinigten organischen Phasen dreimal mit je 100 ml Wasser, einmal mit 100 ml 10%-iger Natronlauge und nochmals mit 100 ml Wasser, trocknet über Natriumsulfat und destilliert. Man erhält 62,9g (55,7% der Theorie) 1-(4-Chlorphenoxy)-2,2-dimethyl-butan-3-on vom Siedepunkt 135-140°C/0,4 Torr.

Vorstufen

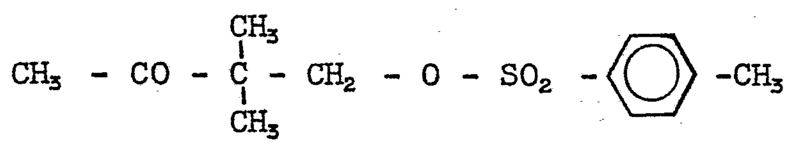

47,6g (0,25 Mol) 4-Toluolsulfochlorid werden in 100 ml Chloroform gelöst, 35g (0,3 Mol) 2,2-Dimethyl-1-hydroxy-butan-3-on zugegeben und bei 0 bis 5°C 40 ml (0,5 Mol) Pyridin zugetropft. Man läßt 15 Stunden bei Raumtemperatur nachrühren, gibt das Reaktionsgemisch auf 200 g Eis und 70 ml konzentrierter Salzsäure, trennt die organische

Phase ab, wäscht sie dreimal mit je 200 ml Wasser nach, trocknet über Natriumsulfat und engt ein. Der Rückstand wird in 100 ml Petrolether aufgenommen, wobei das Endprodukt auskristallisiert. Man erhält 48g (71 % der Theorie) 2,2-Dimethyl-1-tosyloxy-butan-3-on als farblose Kristalle vom Schmelzpunkt 49-52°C.

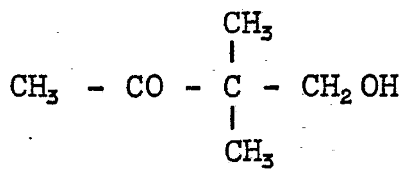

Zu 172g (2 Mol) Methylisopropylketon in 1000 ml Methanol werden 66 g (2,2 Mol) Paraformaldehyd und 1 g Kaliumhydroxid in 10 ml Methanol getropft. Man erhitzt 15 Stunden unter Rückfluß und destilliert anschließend das Methanol über eine Kolonne bei 82°C Innentemperatur ab. Der Rückstand wird im Wasserstrahlvakuum destilliert. Man erhält 152,7g (68 % der Theorie) 2,2-Dimethyl-1-hydroxy-butan-3-on vom Siedepunkt 80-82°C/12 Torr.

Beispiel 2

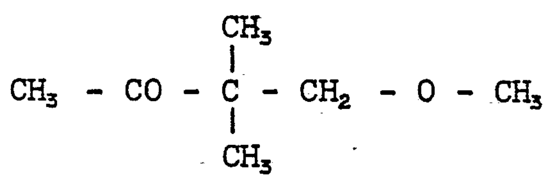

(Verfahren b)

Zu 174g (1,5 Mol) 2,2-Dimethyl-1-hydroxy-butan-3-on (Herstellung vgl. Beispiel 1) und 150 ml (2 Mol) Dimethylsulfat werden bei 40°C (exotherm, leichte Kühlung) 80g (2 Mol) Natriumhydroxid, gelöst in 100 ml Wasser, getropft. Nach beendeter Zugabe läßt man 15 Stunden bei 40°C nachrühren und destilliert das entstandene 2,2-Dimethyl-1-methoxy-butan-3-on mittels Wasserdampf. Die wässrige Phase wird mit Natriumchlorid gesättigt, der ölige

Bestandteil wird abgetrennt und unter vermindertem Druck rektifiziert. Man erhält 123,8g (63 % derTheorie) 2,2-Dimethyl-1-methoxy-butan-3-on vom Siedepunkt 48-62°C/5-8 Torr.

<u>Beispiel 3</u>

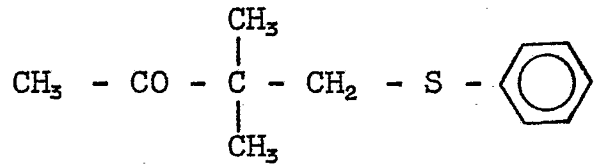

(Verfahren a)

134,5g (1 Mol) 4-Chlorpinakolin werden mit 165g (1,5 Mol) Thiophenol und 210g (L,52 Mol) Kaliumcarbonat in 500 ml Dimethylformamid 15 Stunden bei 150°C und einem Druck von 2 bis 4 bar grührt. Man läßt auf Raumtemperatur abkühlen, verrührt mit 10 l Wasser und extrahiert mit Ether. Die Etherphase wird über Natriumsulfat getrocknet, eingeengt und der Rückstand im Vakuum destilliert. Man erhält 162g(78 % der Theorie) 2,2-Dimethyl-1-phenylmercapto-butan-3-on vom Siedepunkt 112°C/0,5 Torr.

<u>Vorstufe</u>

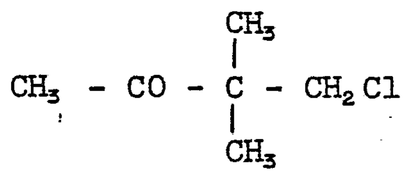

11,6g (0,1 Mol) 2,2-Dimethyl-1-hydroxy-butan-3-on (Herstellung vgl.Beispiel 1) werden bei 50 bis 60°C (Eiskühlung) zu 20,5 g (0,1 Mol) N,N-Diethyl-1,2,2-trichlorvinyl-amin getropft. Nach zweistündigem Rühren bei 60°C wird im Wasserstrahlvakuum destilliert. Man erhält 8,1g (60 % der Theorie) 1-Chlor-2,2-dimethyl-butan-3-on vom Schmelzpunkt 60 - 62°C/12 Torr.

Das 1-Chlor-2,2-dimethyl-butan-3-on wird in einer Ausbeute von 90 % erhalten, wenn man äquimolare Mengen 2,2-Dimethyl-1-hydroxy-butan-3-on und Triphenylphosphin in der zehnfachen Mengen Tetrachlorkohlenstoff 12 Stunden unter Rückfluß erhitzt, das Lösungsmittel abdestilliert, den Rückstand in Ether aufnimmt, filtriert und destilliert).

Beispiel 4

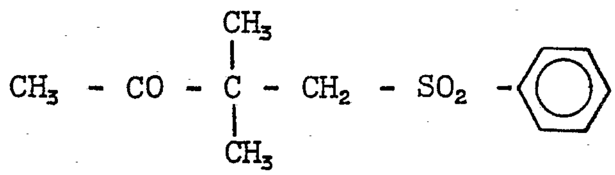

(Verfahren c)

40,6g (0,2 Mol) 2,2-Dimethyl-1-phenylmercapto-butan-3-on (Beispiel 3) werden in 300 ml Eisessig gelöst und mit 30,4g (0,89 Mol) Wasserstoffperoxid versetzt. Man läßt 8 Stunden bei 50°C rühren. Danach wird das Reaktionsgemisch in 250 ml Wasser gegeben und mit Ether extrahiert. Die Etherphase wird mit Natriumhydrogencarbonatlösung und Wasser neutral gewaschen, über Natriumsulfat getrocknet und eingeengt. Man erhält 40,4 g (84,2 % der Theorie)2,2-Dimethyl-1-phenylsulfonyl-butan-3-on vom Schmelzpunkt 56-58°C.

Entsprechend Beispiel 1 bis 4 und entsprechend den erfindungsgemäßen Verfahren (a), (b) und (c) können die nachfolgenden Verbindungen der allgemeinen Formel (I)

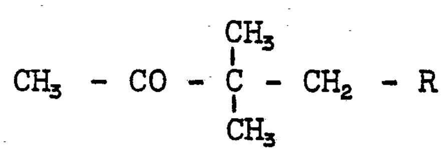 (I)

erhalten werden:

| Bsp.Nr. | R | Siedepunkt(°C)/Torr |
|---|---|---|
| 5 | $-O-C_2H_5$ | 54-58/10 |
| 6 | 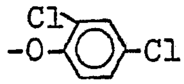 | Oel |
| 7 | 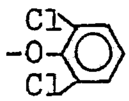 | Oel |
| 8 | 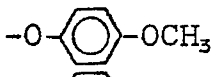 | 90-95/0,15 |
| 9 | 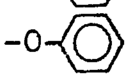 | 83-87/0,15 |
| 10 | $-S-CH_3$ | 80/0,1 |
| 11 | -CN | 85-90/8 |
| 12 | -SCN | 83-87/1 |
| 13 | 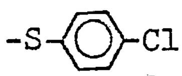 | 146/0,15 |
| 14 | 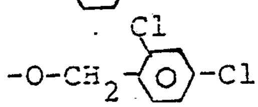 | 115-23/0,03 |
| 15 | 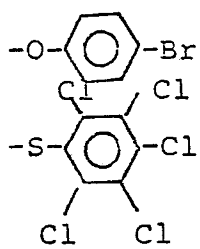 | 96-110/0,4 |
| 16 | | Fp.: 84°C |
| 17 | 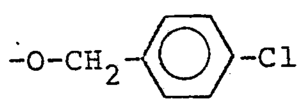 | 123/0,01 |
| 18 | 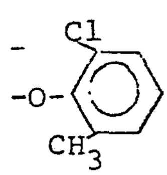 | $n_D^{20}$ = 1,5104 |
| 19 | 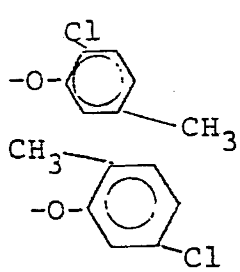 | $n_D^{20}$ = 1,5108 |
| 20 | | $n_D^{20}$ = 1,5103 |

| Bsp.Nr. | R | Siedepunkt(°C)/Torr |
|---|---|---|
| 21 | 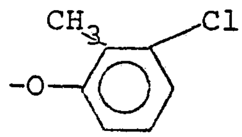 | $n_D^{20}$ = 1,5150 |
| 22 | 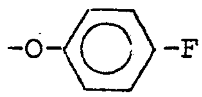 | $n_D^{20}$ = 1,4814 |
| 23 | 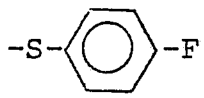 | $n_D^{20}$ = 1,5301 |
| 24 | 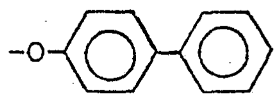 | Fp.= 85 - 87°C |

Folgeprodukte des Beispiels 1

(VI-1) 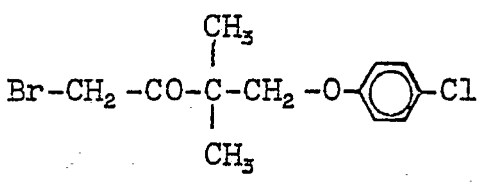

36g (0,159 Mol) 1-(4-Chlorphenoxy)-2,2-dimethyl-butan-3-on (Beispiel 1) werden in 300 ml Chloroform gelöst und bei 20°C tropfenweise so mit 25,5g (0,159 Mol) Brom versetzt, daß laufend Entfärbung eintritt. Nach beendeter Zugabe läßt man 30 Minuten bei Raumtemperatur rühren und engt danach durch Abdestillieren des Lösungsmittels im Vakuum ein. Man erhält 48,5g (quantitativer Umsatz) rohes 1-Brom-4-(4-chlorphenoxy)-3,3-dimethyl-butan-2-on als Oel.

(VII-1) 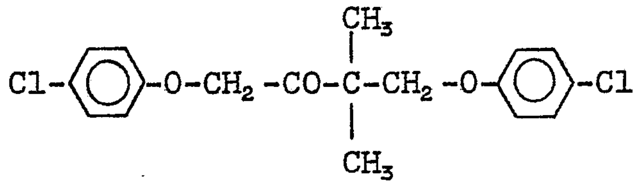

20,6g (0,16 Mol) 4-Chlorphenol und 28g (0,2 Mol) Kaliumcarbonat werden in 250 ml Aceton gelöst. Unter Rückfluß werden langsam 48,5g 1-Brom-4-(4-chlorphenoxy)-3,3-dimethyl-butan-2-on in 50 ml Aceton zugetropft. Nach beendeter Zugabe läßt man 15 Stunden unter Rückfluß rühren, filtriert und engt das Filtrat ein. Der Rückstand wird in 500ml Methylenchlorid aufgenommen, mit 200 ml Wasser , 200 ml gesättigter Natriumhydrogencarbonatlösung und nochmals mit 200 ml Wasser ausgeschüttelt.

Die organische Phase wird über Natriumsulfat getrocknet, eingeengt und der Rückstand in 150 ml Diisopropylether aufgenommen. Nach dem Einengen erhält man 36,6g (65 % der Theorie) 1,4-Bis-(4-chlorpheoxy)-3,3-dimethyl-butan-2-on als farblose Kristalle vom Schmelzpunkt 76-77°c.

(VIII-1) 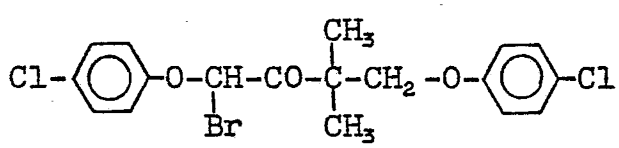

36,6g (o,1 Mol) 1,4-Bis-(4-chlorphenoxy)-3,3-dimethyl-butan-2-on werden in 250 ml Chloroform gelöst und bei 20°C tropfenweise so mit 16,6g (0,1 Mol) Brom versetzt, daß laufend Entfärbung eintritt . Nach beendeter Zugabe läßt man 30 Minuten bei Raumtemperatur nachrühren und engt danach durch Abdestillieren des Lösungsmittels im Vakuum ein. Man erhält 43,2g (quantitativer Umsatz) 1,4-Bis-(4-chlorphenoxy)-1-brom-3,3-dimethyl-butan-2-on als Oel, das direkt weiter umgesetzt wird.

(IX - 1) 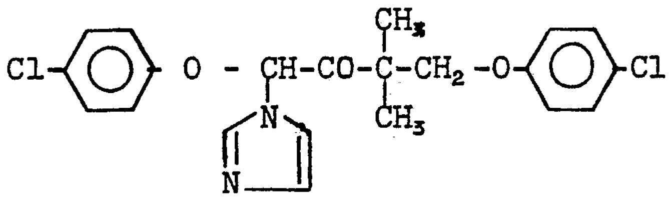

21,6g (0,05 Mol) rohes 1,4-Bis-(4-chlorphenoxy)-1-brom-3,3-dimethyl-butan-2-on werden mit 14 g (0,2 Mol) Imidazol in 100 ml Acetonitril 17 Stunden unter Rückfluß gerührt. Danach wird durch Abdestillieren des Lösungsmittels unter vermindertem Druck eingeengt. Der Rückstand wird in 400 ml Methylenchlorid aufgenommen, dreimal mit je 800 ml Wasser ausgeschüttelt, über Natriumsulfat getrocknet und eingeengt. Der Rückstand wird in 100 ml Aceton aufgenommen und tropfenweise mit 9g (0,05 Mol) 1,5-Naphthalindisulfonsäure-tetrahydrat in 50 ml Aceton versetzt. Der entstehende Niederschlag wird abgesaugt und in 200 ml Methylenchlorid suspendiert. Man gibt 400 ml gesättigte Natriumhydrogencarbonatlösung zu, verrührt 30 Minuten und trennt die organische Phase ab. Nach Abdestillieren des Lösungsmittels erhält man 14,1g (67 % der Theorie) 1,4-Bis-(4-chlorphenoxy)-3,3-dimethyl-1-(imidazol-1-yl)-butan-2-on als Oel.

Patentansprüche

1) 4-Substituierte 3,3-Dimethyl-butan-2-one der allgemeinen Formel

$$CH_3 - CO - \overset{\displaystyle CH_3}{\underset{\displaystyle CH_3}{\overset{|}{\underset{|}{C}}}} - CH_2 - R \qquad (I)$$

in welcher

R für die Gruppierung $-X-R^1$ oder Cyano steht,

$R^1$ für n-Alkyl mit 1 bis 4 Kohlenstoffatomen, Isopropyl, Isobutyl, sek.-Butyl,Alkenyl mit 3 bis 4 Kohlenstoffatomen, Alkinyl mit 3 bis 4 Kohlenstoffatomen, gegebenenfalls substituiertes Aryl oder substituiertes Aralkyl steht, sowie auch für Cyano steht, wenn X für -O- oder -S- steht und

X für -O-, -S-, -SO- oder $-SO_2-$ steht.

2) Verfahren zur Herstellung von 4-substituierten 3,3-Dimethyl-butan-2-onen gemäß Formel I in Anspruch 1, dadurch gekennzeichnet, daß man

a) Butan-2-on-Derivate der Formel

$$\begin{array}{c} \qquad\qquad\quad CH_3 \\ \qquad\qquad\quad | \\ CH_3 - CO - C - CH_2 - Y \\ \qquad\qquad\quad | \\ \qquad\qquad\quad CH_3 \end{array} \qquad (II)$$

in welcher

Y für Chlor, Brom oder die Gruppierung $-O-SO_2-R^2$ steht, wobei

$R^2$ für Alkyl oder gegebenenfalls substituiertes Aryl steht,

mit Verbindungen der Formel

$$R^3 - Me \qquad (III)$$

in welcher

$R^3$ für die Gruppierung $-X^1-R^4$ oder Cyano steht,

$R^4$ für n-Alkyl mit 1 bis 4 Kohlenstoffatomen, Isopropyl,Isobutyl, sek.-Butyl, Alkenyl und Alkinyl mit jeweils 3 bis 4 Kohlenstoffatomen, gegebenenfalls substituiertes Aryl, substituiertes Aralkyl oder Cyano steht,

$X^1$ für Sauerstoff oder Schwefel steht und

Me für ein Alkalimetall oder Wasserstoff steht,

in Gegenwart eines Verdünnungsmittels und gegebenenfalls in Gegenwart eines Säurebindemittels umsetzt, oder

b) 3,3-Dimethyl-4-hydroxy-butan-2-on der Formel

$$CH_3 - CO - \overset{CH_3}{\underset{CH_3}{C}} - CH_2 - O - H \qquad (IV)$$

mit Verbindungen der Formel

$$Y' - R^5 \qquad (V)$$

in welcher

$R^5$ für n-Alkyl mit 1 bis 4 Kohlenstoffatomen, Isopropyl, Isobutyl, sek.-Butyl, Alkenyl und Alkinyl mit jeweils 3 bis 4 Kohlenstoffatomen oder substituiertes Aralkyl steht,

$Y'$ für Chlor, Brom oder die Gruppierung $-O-SO_2-R^6$ steht und

$R^6$ für Alkyl, Alkoxy oder gegebenenfalls substituiertes Aryl steht,

gegebenenfalls in Gegenwart eines Verdünnungsmittels und gegebenenfalls in Gegenwart eines Säurebindemittels umsetzt, und

c) gegebenenfalls die nach dem Verfahren (a) erhältlichen Verbindungen der Formel

$$CH_3 - CO - \overset{CH_3}{\underset{CH_3}{C}} - CH_2 - S - R^7 \qquad (Ia)$$

in welcher

$R^7$ für n-Alkyl mit 1 bis 4 Kohlenstoffatomen, Isopropyl, Isobutyl, sek.-Butyl, Alkenyl und Alkinyl mit jeweils 3 bis 4 Kohlenstoffatomen, gegebenenfalls substituiertes Aryl oder substituiertes Aralkyl steht,

in üblicher Weise mit einem Oxidationsmittel umsetzt.

3) Verwendung von 4-substituierten 3,3-Dimethyl-butan-2-onen als Zwischenprodukte zur Herstellung von Pflanzenschutzmitteln mit fungizider Wirkung.

0041671

Europäisches Patentamt

# EUROPÄISCHER RECHERCHENBERICHT

Nummer der Anmeldung

EP 81 10 4158.1

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | JOURNAL OF ORGANIC CHEMISTRY, Band 42, Nr. 10, 13. Mai 1977<br>Easton<br>H.O. HOUSE et al. "Synthesis of ω - Bromo Ketones"<br>Seiten 1709 bis 1717<br>* Seite 1710, Formeln 11, 15 *<br>-- | 1 | C 07 C 49/175<br>C 07 C 49/213<br>C 07 C 49/233<br>C 07 C 49/255<br>C 07 C 119/042<br>C 07 C 149/10<br>C 07 C 149/30<br>C 07 C 149/34 |
| A,D | DE - A1 - 2 635 666 (BAYER)<br>* Ansprüche 1, 3 bis 6 *<br>---- | 3 | |
| | ./.. | | |

RECHERCHIERTE SACHGEBIETE (Int. Cl.³)

A 01 N 43/50
C 07 C 49/175
C 07 C 49/213
C 07 C 49/233
C 07 C 49/255
C 07 C 119/042
C 07 C 147/06
C 07 C 149/10
C 07 C 149/30

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 17-09-1981 | KNAACK |

Nummer der Anmeldung

Europäisches Patentamt

# EUROPÄISCHER RECHERCHENBERICHT

EP 81 10 4158.1


| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | | | C 07 C 147/06<br>C 07 C 161/04<br>C 07 D 233/58<br>A 01 N 43/50 |
| | | | RECHERCHIERTE SACHGEBIETE (Int. Cl.³) |
| | | | C 07 C 149/34<br>C 07 C 161/04<br>C 07 D 233/58 |